# EUROPÄISCHE PATENTSCHRIFT

(11) **EP 2 032 690 B1**
(45) Veröffentlichungstag und Bekanntmachung des Hinweises auf die Patenterteilung: **13.06.2012**
(21) Anmeldenummer: 07725549.5
(22) Anmeldetag: 25.05.2007
(51) Int. Cl.: C12N 5/071, C12M 3/08

(54) **VERFAHREN ZUR IN VITRO PRÄPARATION VON LEBENDEN ZELLEN UND/ODER ZELLANSAMMLUNGEN AUS EINEM GEWEBE**
METHOD FOR THE IN VITRO PREPARATION OF LIVING CELLS AND/OR CELL ACCUMULATIONS FROM TISSUE
PROCÉDÉ DE PRÉPARATION IN VITRO DE CELLULES VIVANTES ET/OU D'ACCUMULATIONS DE CELLULES PROVENANT D'UN TISSU

(30) Priorität: 29.05.2006 DE 102006026179
(43) Veröffentlichungstag der Anmeldung: 11.03.2009
(73) Patentinhaber: NMI Naturwissenschaftliches und Medizinisches Institut an der Universität Tübingen, 72770 Reutlingen (DE)
(72) Erfinder: LEMBERT, Nicolas, 72108 Rottenburg (DE)
(74) Vertreter: Witte, Weller & Partner
(86) Internationale Anmeldenummer: PCT/EP2007/004653
(87) Internationale Veröffentlichungsnummer: WO 2007/137769

(56) Entgegenhaltungen:
- EP-A- 0 236 049
- LEMBERT N ET AL: "Areal density measurement is a convenient method for the determination of porcine islet equivalents without counting and sizing individual islets." CELL TRANSPLANTATION, Bd. 12, Nr. 1, 2003, Seiten 33-41, XP009092274 ISSN: 0963-6897
- ROBERTSON K G S M ET AL: "The optimization of large-scale density gradient isolation of human islets" ACTA DIABETOLOGICA, Bd. 30, Nr. 2, 1993, Seiten 93-98, XP009092371 ISSN: 0940-5429
- MCMANNIS ET AL: "Chapter 7: Use of the COBE 2991 cell processor fpr mone marrow processing" BONE MARROW PURGING AND PROCESSING, XX, XX, 1991, Seiten 73-85, XP001536591
- GILMORE M J M L ET AL: "A TECHNIQUE FOR RAPID ISOLATION OF BONE MARROW MONONUCLEAR CELLS USING FICOLL-METRIZOATE AND THE IBM 2991 BLOOD CELL PROCESSOR" BRITISH JOURNAL OF HAEMATOLOGY, OXFORD, GB, Bd. 50, Nr. 4, 1982, Seiten 619-626, XP009040372 ISSN: 0007-1048
- GRAZIANO J H ET AL: "A SIMPLE TECHNIQUE FOR PREPARATION OF YOUNG RED CELLS FOR TRANSFUSION FROM ORDINARY BLOOD UNITS" BLOOD, Bd. 59, Nr. 4, 1982, Seiten 865-868, XP009092554 ISSN: 0006-4971
- RICORDI C ET AL: "A method for the mass isolation of islets from the adult pig pancreas." DIABETES JUN 1986, Bd. 35, Nr. 6, Juni 1986 (1986-06), Seiten 649-653, XP009092278 ISSN: 0012-1797
- RICORDI CAMILLO ET AL: "Clinical islet transplantation: advances and immunological challenges." NATURE REVIEWS. IMMUNOLOGY APR 2004, Bd. 4, Nr. 4, April 2004 (2004-04), Seiten 259-268, XP009092270 ISSN: 1474-1733
- KLAFFSCHENKEL R A ET AL: "A closed system for islet isolation and purification using the COBE2991 cellprocessor may reduce the need of clean room facilities" CELL TRANSPLANTATION, ELSEVIER SCIENCE, US, Bd. 16, Nr. 6, 2007, Seiten 587-594, XP009092273 ISSN: 0963-6897

## Beschreibung

Die vorliegende Erfindung betrifft ein Verfahren zur *in vitro* Präparation von lebenden Zellen und/oder Zellansammlungen aus einem Gewebe, mit mehreren Schritten, wobei in einem Schritt ein Zellprozessor verwendet wird.

Die Bereitstellung von lebenden Zellen oder Zellansammlungen spielt in der heutigen Medizin eine immer wichtigere Rolle, da die isolierten lebenden Zellen bspw. als somatische Zelltherapeutika zur Behandlung verschiedenster Krankheiten eingesetzt werden können.

So sind beispielsweise Zellen von Interesse, die aus Pankreas, Leber, Herz, Niere, Lunge, Blase, Ösophagus, Darm, Epidermis, Knorpel etc., grundsätzlich aus allen Geweben oder Organen isoliert werden können. Diese Zellen/Zellansammlungen werden gesunden Organen und/oder Geweben von Spendern entnommen, und in Patienten mit einem Bedarf einer Therapie des erkrankten, korrespondierenden Organs/Gewebes transplantiert.

So ist im Stand der Technik bspw. bekannt, Langerhans-Inseln zu isolieren, und die isolierten Inseln zur Therapie von Diabetes mellitus einzusetzen. Diese Krankheit ist mit einem Mangel an Insulin verbunden, das die Glukoseabsorption aus dem Blut durch Einwirken auf die peripheren Organe bewirkt. Fehlt das Insulin, können die peripheren Organe die Glukose im Blut nicht verbrauchen, wodurch der Glukose-Level im Blut sehr hoch bleibt. Dieser Zustand wird als Diabetes bezeichnet. Der Typ-1-Diabetes, auch als juveniler Diabetes bezeichnet, ist ein Krankheitszustand, bei welchem die Pankreas-Zellen aus bestimmten Gründen zerstört und kein Insulin sezenrniert wird. Beim Typ-2-Diabetes besitzt das Insulin - obwohl es in normalen oder höheren Konzentrationen im Blut vorliegt - eine niedrigere Aktivität als das Insulin von gesunden Menschen. Dies ist auf eine Insulin-Resistenz in den peripheren Organen zurückzuführen.

Aus nahe liegenden Gründen wird daher die Transplantation von Langerhans-Inseln derzeit nur bei Diabetes vom Typ 1 eingesetzt. Wie bereits vorstehend erwähnt, wird der Typ-1-Diabetes durch eine fehlgeleitete Immunantwort verursacht, die die Insulin produzierenden Pankreas-Zellen spezifisch zerstört. Als Therapie können daher entweder der Pankreas selber oder aber Langerhans-Zellen implantiert werden. Da die Transplantation des gesamten Organs jedoch oftmals mit Komplikationen und außerdem mit einem hohen Aufwand verbunden und riskikobehaftet ist, stellt die Transplantation von lediglich der Langerhans-Inseln eine minimal-invasive und daher in den meisten Fällen die optimalere Therapie dar.

Gleiches gilt für Zellarten oder Zellansammlungen aus anderen Geweben oder Organen. Durch die Transplantation von lediglich den funktionsfähigen Zellen/Zellansammlungen wird die Transplantation ganzer Organe und die damit verbundenen Umstände und Komplikationen vermieden.

Da die zu transplantierenden Zellen für eine Transplantation unbedingt lebens- und funktionsfähig sein müssen, besteht in den einschlägigen medizinischen Gebieten ein großer Bedarf für Verfahren, mit welchen die Zellen so isoliert werden können, dass sie ihre natürliche Funktion beibehalten. Solche Isolationsverfahren sind daher die Voraussetzung für eine erfolgreiche Transplantation und Zelltherapie.

Prinzipiell wird für eine Zellisolierung zunächst das gewünschte Gewebe oder Organ einem Spender entnommen und dieses anschließend beispielsweise enzymatisch verdaut und/oder mechanisch behandelt, wodurch das Gewebe/Organ entsprechend abgebaut/zerkleinert wird. Dabei ist darauf zu achten, dass die im Gewebe/Organ enthaltenden Zellen von Interesse intakt bleiben. In abschließenden Schritten werden die Zellen von Interesse durch gezielte Isolierungsschritte gereinigt und ggf. vereinzelt.

Im Stand der Technik sind derartige Verfahren beispielsweise zur Isolierung der Langerhans-Inseln aus dem Pankreas bekannt. Diese Techniken basieren dabei auf dem von Dr. Ricordi entwickelten Verfahren. Dieses Verfahren besteht aus den folgenden Schritten: einer Volumenvergrößerung des Pankreas unter Verwendung einer Kollagenaselösung, einem Verdau des Pankreas-Gewebes, sowie der Reinigung und Isolierung der in dem verdauten Material vorliegenden Inseln. Zur Reinigung der Inseln wird gewöhnlich ein Zellprozessor, wie bspw. der COBE2991 verwendet.

Das bekannte Isolierungs-Verfahren beginnt - nach Organentnahme - mit der Infusion der Kollagenaselösung in den Hauptgang des Pankreas, um diesen zu expandieren, wobei sich die Lösung durch das Pankreasgewebe hindurch verbreitet. Der expandierte Pankreas wird anschließend in eine sogenannte Ricordi-Kammer überführt, deren Leitungen ebenfalls mit der Lösung gefüllt sind und die auf Körpertemperatur erwärmt ist. Dadurch wird die in das Pankreas eingeführte (kalte) Kollagenaselösung aktiviert und löst die Kollagenfasern auf, die das Pankreas-Gewebe verbinden.

Dieser Schritt des Verdauens muss sorgfältig beobachtet werden, da bei einem Überverdau die Zellenansammlungen, die die Inseln bilden, in einzelne Zellen dissoziieren. Eine Dissoziierung der Inseln ist im Hinblick auf eine Transplantation der Zellen nicht vorteilhaft, da die Inseln - wenn sie in ihre Zellen vereinzelt sind - schwierig zu reinigen und zu isolieren sind, und da ferner die zellulären Aktivitäten der Inseln beschädigt werden können.

Der Verdau wird durch Einführen einer Lösung mit Raumtemperatur gestoppt, und das Pankreas-Gewebe anschließend durch eine Zentrifugation gesammelt. Die gesammelten Gewebebestandteile werden in einem letzten Schritt der Reinigung beispielsweise durch eine kontinuierliche Dichtegradienten-Zentrifugation gereinigt, wobei im Stand der Technik hier beispielsweise ein COBE2991-Zellprozessor und eine Dextranlösung mit niedrigem Molekulargewicht eingesetzt werden. Aufgrund ihrer Dichte verteilen sich die Gewebebestandteile, darunter die Inseln und das exokrine Pankreas-Gewebe, in diesem Dichtegradienten in unterschiedliche Dichtebanden.

Anschließend werden die Gradienten-Fraktionen auf das Vorliegen der Inseln kontrolliert und die entsprechende(n) Fraktion(en) gesammelt.

Eine Übersicht über das gegenwärtig übliche Reinigungs- und Isolationsverfahren bietet beispielsweise der Artikel "Clinical Islet Transplantation: Advances and Immunological Challenges" Ricordi und Strom, Nature Reviews, Immulogy (2004) 4: 259-268.

Wie aus dem Verfahrensablauf zu erkennen ist, wird während der Präparation der Langerhans-Inseln für die Herstellung somatischer Zelltherapeutika mehrmals am offenen Zellsystem gearbeitet. Um die GMP-Richtlinien (Good Manufactoring Practices) zur Herstellung somatischer Zelltherapeutika einzuhalten, muss daher nicht nur die Organentnahme selber, sondern auch die gesamte nachfolgende Präparation der Zellen/Zellansammlungen in einem Reinraumlabor durchgeführt werden. Diese Maßnahmen sind mit einem großen Aufwand verbunden, sowohl was den personellen und den apparativen Aufwand angeht, als auch was die Kosten zur Finanzierung dieser Maßnahmen betrifft.

Aufgabe der vorliegenden Erfindung ist es daher, ein neues Verfahren zur Präparation von lebenden Zellen und/oder Zellansammlungen aus einem Gewebe/Organ bereitzustellen, mit welchem der personelle, apparative und finanzielle Aufwand gegenüber den herkömmlichen Verfahren deutlich gesenkt, und das Gesamtverfahren beschleunigt werden kann.

Gemäß der vorliegenden Erfindung wird diese Aufgabe durch die Bereitstellung eines Verfahrens mit den folgenden Schritten gelöst:
a) Bereitstellen von Gewebe- und/oder Organmaterial, das die Zellen von Interesse aufweist,
b) Behandeln des Gewebes in einem Behältnis zur Gewinnung von zumindest teilweise zerlegtem und/oder abgebautem Gewebe- und/oder Organmaterial,
c) Hinzufügen von flüssigem Medium zu dem Gewebe- und/oder Organmaterial aus Schritt b), um zumindest Teile des Gewebe- und/oder Organmaterials aus dem Behältnis in einen Beutel eines Zellprozessors zu eluieren, wobei das Behältnis und der Beutel derart verbunden sind, dass sie ein geschlossenes System bilden,
d) Zentrifugieren des Beutels und Verwerfen des Überstandes zur Gewinnung eines die Zellen von Interesse enthaltenden Konzentrats in dem Zellprozessor,
e) Reinigen des Konzentrats unter Anwendung eines Dichteradienten in dem Zellprozessor.

Die der Erfindung zugrunde liegende Aufgabe wird auf diese Weise vollkommen gelöst.

Mit der vorliegenden Erfindung wird ein Verfahren bereitgestellt, bei dem außer der initialen Organpräparation alle Schritte zur Zellgewinnung innerhalb eines geschlossenen, keimfreien Schlauch- und Beutelsystems durchgeführt werden können. Durch diese Präparations- und Verfahrenstechnik ist es möglich, eine aseptische Zellpräparation nach GMP-Kriterien außerhalb eines Reinraums durchzuführen.

Durch das Verfahren wird eine Technik bereitgestellt, die deutlich schneller und billiger als die im Stand der Technik bekannten Verfahren ist, und ferner wesentlich weniger Ressourcen voraussetzt als die bekannte Reinraumpräparation.

Bei dem erfindungsgemäßen Verfahren wird also Gewebe oder ein Organ bereitgestellt, das die Zellen/Zellansammlungen von Interesse enthält. Das Gewebe/Organ wird in einem Behältnis gegeben und zur Gewinnung von zumindest teilweise zerkleinertem und/oder abgebauten Gewebematerial behandelt.

Dabei ist in einer Ausführungsform bevorzugt, wenn die Behandlung des Gewebes in Schritt b) durch zumindest ein Enzym erfolgt. Das Enzym ist dabei vorteilhafterweise ausgewählt aus der Gruppe der Kollagenasen, insbesondere Liberase oder Kollagenase/Protease oder Mischungen davon.

In einem nächsten Schritt wird zu dem Gewebematerial in dem Behältnis flüssiges Medium hinzugefügt, wobei zumindest Teile des behandelten Gewebematerials aus dem Behältnis in einen Beutel eines Zellprozessors eluiert werden. Dabei sind das Behältnis und der Beutel derart verbunden, dass sie ein geschlossenes System bilden, wodurch das Gewebematerial/Zellen enthaltende Eluat, das vom Behältnis in den Beutel überführt wird, dadurch nicht mit der Atmosphäre in Kontakt kommt. In einem nächsten Schritt wird der Beutel in dem Zellprozessor zentrifugiert und anschließend der Überstand durch zur Gewinnung eines die Zellen von Interesse enthaltenden Konzentrats in dem Zellprozessor entfernt. Das Entfernen des Überstandes wird dabei durch Zellprozessor-interne Abläufe/Mechanismen durchgeführt, wobei wiederum sichergestellt ist, dass der Inhalt des Beutels nicht mit der Atmosphäre in Kontakt kommt. Schlussendlich wird das im Beutel verbleibende Konzentrat unter Anwendung eines Dichteradienten in dem Zellprozessor gereinigt. Die Gradienten-Lösungen werden dabei ebenfalls unter Ausschluss des Atmosphärenkontakts in den Beutel gegeben. Aufgrund Ihrer unterschiedlichen Dichten verteilen sich die in dem Gewebematerial vorliegenden Zellen oder Gewebebestandteile in dem Gradienten, so dass sich Zellen mit gleicher Dichte (also vorwiegend Zellen vom gleichen Typus) in bestimmten Fraktionen sammeln und so gezielt isoliert werden können.

Bei dem neuen Verfahren wird somit der Zellprozessor vorteilhafterweise als Zentrifuge zur Konzentration eingesetzt, und nicht lediglich - wie im Stand der Technik - zur Reinigung des Konzentrats. Dadurch wird der direkte Atmosphärenkontakt der Zellen während der Präparation vermieden.

Im Stand der Technik werden üblicherweise die Schritte zur Gewebekonzentration und zur Elution des Gewebematerials in einer zweiten Raumzentrifuge außerhalb des Zellprozessors durchgeführt. Bei den im Stand der Technik bekannten Verfahren werden die Reinräume also nicht nur für die anfängliche Organpräparation benötigt, sondern auch für die wiederholten Zentrifugationsschritte, mit welchen die große Menge an Elutionsmedium in einer Raumzentrifuge konzentriert wird, bevor das Konzentrat in einem Zellprozessor über einen Dichtegradienten gereinigt wird. Der Zellprozessor wurde bisher also immer lediglich zur Reinigung eines verschiedene Zellen enthaltendes Konzentrats eingesetzt.

Der Erfinder des vorliegenden Verfahrens hat in eigenen Versuchen erkannt, dass vorteilhafterweise alle Schritte zur Gewebekonzentration und -reinigung innerhalb eines Beutels des Zellprozessors durchgeführt werden können.

Mit diesem Verfahren konnte der Zeitaufwand der Zellpräparation deutlich gesenkt werden, so dass die gesamte Präparation durch zwei Personen innerhalb von vier Stunden vollständig durchgeführt werden konnte, was eine Verdoppelung der Verfahrensgeschwindigkeit bedeutet.

Gemäß der vorliegenden Erfindung wird der Zellprozessor vorteilhafterweise also nicht nur zur Reinigung der Zellen, sondern auch zur Konzentration des Elutionsmediums eingesetzt, wobei die Konzentrationsschritte und Reinigungsschritte im gleichen Beutel des Zellprozessors durchgeführt werden.

Es versteht sich, dass das erfindungsgemäße Verfahren sowohl für eine Präparation von humanen Zellen/Zellansammlungen angewandt werden kann, als auch für die Präparation von tierischen Zellen/Zellansammlungen, also insbesondere allgemein für Zellen/Zellansammlungen von Säugetieren.

Bei einer Weiterbildung des erfindungsgemäßen Verfahrens ist bevorzugt, wenn die Schritte c) und d) vor Durchführung des Schritts e) wiederholt werden.

Bei dieser Ausführungsform ist von Vorteil, dass das gesamte Elutionsmedium prozessiert werden kann, das heißt sämtliches Zellmaterial, das im Elutionsmedium vorliegt, kann konzentriert werden.

Bei einer Weiterführung des erfindungsgemäßen Verfahrens ist bevorzugt, wenn das Behältnis, in welches das Gewebe zur Behandlung gemäß Schritt b) gegeben wird, über eine Kühlvorrichtung mit dem Beutel des Zellprozessors verbunden ist.

Auch bei dieser Ausführungsform ist gewährleistet, dass das Behältnis mit dem Beutel über die Kühlvorrichtung ein geschlossenes System bildet, wodurch das Gewebematerial nicht mit Atmosphärenluft in Kontakt kommt. Durch das Vorsehen einer Kühlvorrichtung wird bspw. im Falle eines Einsatzes von temperaturabhängigen Enzymen in Schritt b) sichergestellt, dass das Enzym inaktiviert wird.

Insgesamt ist bevorzugt, wenn der Zellprozessor ein COBE2991-Zellprozessor ist.

Der COBE2991-Zellprozessor wird von der Firma Gambro BCT vertrieben; eine erste Beschreibung dieses Zellseparators findet sich bspw. in Lake et al., "Large-Scale Purification of Human Islets Utilizing Discontinuous Albumin Gradient on IBM 2991 Cell Separator", Diabetes 1989; 38: 143-145. Im Übrigen wird auf die Betriebsanleitung des Herstellers (Gambro) verwiesen.

In einer Ausführungsform ist bevorzugt, wenn das Medium, das in Schritt c) hinzugefügt wird, ausgewählt ist aus der Gruppe umfassend isoosmolare oder nahezu isoosmolare Lösungen, insbesondere HBSS-Puffer, klinisch zugelassene Infusionslösungen, physiologische Kochsalzlösung, Ringer-Lösung, Ringer-Laktat-Lösung, Lösungen, die in ihrer Zusammensetzung dem Extrazellularraum entsprechen, oder Mischungen davon. Möglich sind auch Zellkulturlösungen wie HAMS, MEM und ähnliche, sowie Zell-Konservierungslösungen, die aufgrund ihrer Ionenzusammensetzung dem Intrazellularraum entsprechen, wie bspw. Eurocollins Lösung, oder Organ-Konservierungslösungen, wie Belzers UW-Lösung, HTK, oder Celsior, oder Mischungen davon, sowie hyperosmolare Lösungen, wie bspw. Pancoll oder Biocoll, sowie Mischungen davon.

Insbesondere ist bevorzugt, wenn bei dem Verfahren ein Gewebe bereitgestellt wird, das ausgewählt ist aus der Gruppe umfassend Pankreas, Leber, Herz, Niere, Lunge, Blase, Ösophagus, Darm, Epidermis, Knorpel.

Prinzipiell kann bei dem erfindungsgemäßen Verfahren jedes Organ bzw. jedes Gewebe eingesetzt werden, aus welchem Zellen oder Zellansammlungen von Interesse derart isoliert werden sollen, dass sie als Zelltherapeutika eingesetzt werden können. Es versteht sich, dass das Organ ein tierisches oder menschliches Organ/Gewebe sein kann.

Insbesondere ist bevorzugt, wenn Zellen und/oder Zellansammlungen präpariert werden, die ausgewählt sind aus der Gruppe umfassend Langerhans-Inseln/Inselzellen, duktale Pankreaszellen/ Ausführgänge, exokrines Pankreasgewebe; Hepatozyten, Cardiomyozyten, Nierenparenchym, Epitelzellen/Mukosazellen, Chondrozyten, Muskelzellen aus der Darmwand und aus der Blasenwand.

Mit dem erfindungsgemäßen Verfahren können demnach die aufgeführten Zellarten, jedoch im Prinzip jede beliebige in einem Gewebe oder einem Organ vorliegende Zellart, aseptisch präpariert werden. Insbesondere ist auch die Isolierung von Stammzellen aus einem Zelllysat möglich, welche beispielsweise in viszeralen Organen oder in den oben aufgeführten Geweben vorliegen. Die Isolierung von Stammzellen ist insbesondere interessant, da diese sich in verschiedene Zellarten differenzieren lassen und daher per se für einen breiten Therapie-Einsatz geeignet sind. Die derart isolierten Zellen bzw. Zellansammlungen können dann für eine somatische Zelltherapie eingesetzt werden, und Patienten mit einem Mangel an diesen Zellen, bzw. mit Defekten in diesen Zellen, verabreicht werden.

Es versteht sich, dass mit dem erfindungsgemäßen Verfahren jede beliebige Zellart aus jedem beliebigen Gewebe isoliert werden kann, egal ob das Organ/Gewebe tierischer oder humaner Herkunft ist. So kann bspw. praktisch jedes Säugetier an Diabetes erkranken, so dass das erkrankte Tier durch entsprechende Zelltherapeutika behandelt werden kann, wobei die den Zelltherapeutika zugrunde liegenden Zellen durch das erfindungsgemäße Verfahre präpariert werden.

Bei einer Ausführungsform des erfindungsgemäßen Verfahrens ist bevorzugt, wenn der enzymatische Verdau des Gewebes in Schritt b) und das Hinzufügen von Medium in Schritt c) in einer Ricordi-Kammer durchgeführt wird.

Die Ricordi-Kammer wird gegenwärtig und auch in dem vorliegend beanspruchten Verfahren als Behältnis eingesetzt, um das entsprechende Gewebe, beispielsweise das Pankreasgewebe, zu verdauen. Die Leitungen der Ricordi-Kammer sind dabei mit einer Kollagenaselösung gefüllt und die Kammer auf Körpertemperatur erwärmt. Bei dieser Temperatur wird das Enzym in der Lösung aktiviert, wodurch das Pankreasgewebe verdaut wird. Zu dem so behandelten Gewebe wird Elutionsmedium gegeben, wodurch zumindest ein Teil des verdauten Gewebematerials aus der Kammer herausfiltriert wird. In der Ricordi-Kammer wird also kontinuierlich verdaut und eluiert/filtriert, wobei das Eluat/Filtrat bspw. über eine Kühlvorrichtung direkt und ohne Atmosphärenkontakt in einen Beutel eines Zellprozessors überführt wird.

Im Stand der Technik wird die Ricordi-Kammer üblicherweise immer über eine Sammelleitung an einer Sammelflasche angeschlossen, in welche das Eluat/Filtrat geführt wird. Anschließend werden die in dieser Sammelflasche gesammelten Inseln oder die Zellen von Interesse anschließend in eine erste Zentrifuge überführt, um das Material zu konzentrieren. Das Konzentrat wird dann vom Überstand getrennt und in eine neue Zentrifuge, bzw. in einen Zellprozessor überführt, so dass bei dem im Stand der Technik bekannten Verfahren zum einen verschiedene Apparaturen als auch mehrere Handgriffe mehrerer Personen notwendig waren, die sämtlich in einem Reinraum vorgenommen werden mussten.

Bei dem erfindungsgemäßen Verfahren wird die Ricordi-Kammer über entsprechende Leitungen und ggf. Kühlvorrichtungen direkt mit dem Zellprozessor, bzw. mit einem Beutel des Zellprozessors, verbunden, so dass das aus der Kammer in den Beutel zu überführende Material keinem Atmosphärenkontakt ausgesetzt ist. Dadurch werden vorteilhafterweise unnötige Apparaturen, insbesondere Zentrifugen, vermieden sowie zusätzliche Handgriffe durch weiteres Personal erspart.

In einer weiteren Ausführungsform ist bevorzugt, wenn für die Dichtegradienten-Reinigung in Schritt e) ein diskontinuierlicher Dichtegradient eingesetzt wird, wobei die Dichtegradienten-Lösungen insbesondere ausgewählt sind aus der Gruppe umfassend, UW (University of Wisconsin), UW/OptiPrep, Ficoll, Sucrose, Percoll, Albumin, Dextran, Iodixanol, oder Mischungen davon. Die aufgeführten Medien können auch in einem kontinuierlichen Gradienten verwendet werden.

Mit den Lösungen wird (in dem Beutel des Zellprozessors) ein Gradient aufgebaut, wobei hierfür Lösungen mit unterschiedlichen, immer kleiner werdenden Dichten übereinander geschichtet werden. Die dissoziierten Zellen werden dann durch Zentrifugieren aufgrund ihrer Schwebedichte unter dem Einfluss starker Zentrifugalkräfte sortiert.

Im Rahmen der vorliegenden Erfindung kann ein COBE2991-Zellprozessor zur Elution und Konzentration von lebenden Zellen aus einem Gewebe verwendet werden. In diesem Zusammenhang werden die Eigenschaften des COBE2991-Zellprozessors nicht nur zur Reinigung von bereits konzentrierten Zellen eingesetzt, sondern eben auch zur Konzentration dieser Zellen. Durch die neue Verwendung kann der Zellprozessor gleichzeitig für mindestens zwei Schritte eingesetzt werden, was im Stand der Technik bisher nicht bekannt war.

Ferner betrifft die vorliegende Erfindung ein Set zur *in vitro Präparation* von lebenden Zellen und/oder Zellansammlungen aus einem Gewebe und/oder Organ, umfassend eine Ricordi-Kammer, eine Kühlvorrichtung und einen Zellprozessor, wobei die Ricordi-Kammer über die Kühlvorrichtung mit dem Zellprozessor derart verbunden ist, dass die Ricordi-Kammer und der Zellprozessor ein geschlossenes System bilden, sowie ggf. dazu notwendige Leitungen und Thermostate. Beispielsweise kann ein COBE2991-Zellprozessor eingesetzt werden.

Mit diesem erfindungsgemäßen Set wird ein Mittel bereitgestellt, mit dem Zellen *in vitro* für eine somatische Zelltherapie einfach und in wenigen Schritten isoliert und gereinigt werden können, ohne dass hierfür ein Reinraum notwendig ist. Der Reinraum wird dann lediglich für die initiale Präparation der verwendeten Organe/Gewebe benötigt.

Die Erfindung wird anhand des nachstehenden Beispiels näher erläutert.

### Beispiel

### Material und Methoden

### Organgewinnung

Sämtliche Organe wurden sechs Monate alten Schweinen entnommen. Die Bauchspeicheldrüsen wurden sofort nach Entnahme von Fett, Gefäßen und Bindegewebe gereinigt. Der Milzlappen der Bauchspeicheldrüse wurde über den Pankreas-Gang mit 50 ml eiskalter UW (Universtiy of Wisconsin)-Lösung zum Schutz des Organs vor Ischämieschäden oder Selbstverdauung infundiert.

### Pankreasverdauung

Über den Pankreas-Gang wurde in den Milzlappen jeweils Liberase (Roche, 1,5 mg/g Gewebe, 150 ml) injiziert. Die Organe wurden in einer handgefertigten, mit einem Heizbad (400 ml) verbundenen Ricordi-Kammer (manuelles Schütteln, Volumen 600 ml, Maschengröße 600 µm, 8 rostfreie Stahlkugeln). Vor dem Verdau wurde das System auf 37°C mit einem Liter HBSS (Biochrom AG, Berlin, Deutschland) aufgewärmt, wobei diese Lösung mit HEPES (Biochrom AG, Berlin, Deutschland 25 mM) versetzt war.

### Geschlossenes System zur Isolierung und Reinigung der Langerhans-Inseln

Das geschlossene System für die Isolierung und Reinigung de Inseln bestand aus der Ricordi-Kammer für das kontinuierliche Verdauen/Filtrieren, einer Kühlvorrichtung aus rostfreien Behältern und einem COBE2991-Zellprozessor. Mit diesem Apparateaufbau wurde vermieden, das verdaute Gewebematerial in vorgekühlten Zentrifugationsbehältem sammeln und eine zweite Raumzentrifuge einsetzen zu müssen.

Nach der optimalen Organ-Dissoziierung in der Ricordi-Kammer wurde die Phase des kontinuierlichen Verdauens/Filtrierens gestoppt, neues Medium für die Elution hinzugefügt, und das verdaute Material über die Kühlvorrichtung direkt in einen Beutel des COBE2991-Zellprozessors überführt (Gambro BCT, Martinsried, Deutschland). Ein Maximum von 600 ml Verdau (10°C) wurde sofort für eine Minute und 1.500 U/min zentrifugiert. Über die "Super-out-Funktion" des Zellprozessors wurde der Überstand aus dem Beutel vollständig entfernt, wodurch ein konzentrierter Verdau (50 ml) in dem Beutel zurückblieb. Der Überstand (14°C) wurde auf die Abwesenheit von Inseln kontrolliert. Die Ricordi-Kammer wurde anschließend mit frischem HBSS-Puffer (Raumtemperatur, 500 ml) versetzt, wodurch wieder Gewebematerial von der Kammer über die Kühlvorrichtung in den Beutel eluiert wird. Das gekühlte Eluat wurde dann wiederum im COBE-Zellprozessor zentrifugiert. Dieses Verfahren wurde solange wiederholt, bis insgesamt drei Liter Puffer prozessiert wurden, und sämtliches verdautes Gewebematerial als Konzentrat im Beutel vorlag. Aus dem konzentrierten Verdau in dem Beutel wurden die Inseln unter Verwendung eines modifizierten diskontinuierlichen UW/OptiPrep-Gradienten gereinigt: Eine Grundschicht (UW/OptiPrep-Lösung, Dichte 1,2: Progen, Heidelberg, Deutschland) wurde hinzugefügt und für einen Zeitraum von 15 Minuten gemischt, um das konzentrierte Gewebe ("Misch-Funktion, COBE2991") aufzulösen. Anschließend wurde eine mittlere Schicht (UW/OptiPrep-Lsöung, 100 ml Dichte 1,09), gefolgt von einer Deckschicht (UW-Lösung, 120 ml Dichte 1,05) hinzugefügt. Nach einer Zentrifugation (1.000 U/min, 5 Minuten) wurde das Gewebe abgepumpt und Fraktionen (20 ml) gesammelt, die hinsichtlich des Vorliegens von Inseln untersucht wurden. Die Insel-Ausbeute wurde durch Dichteanalysen wie zuvor beschrieben bestimmt (siehe Lembert et al., "Areal Density Measurement Is a Convenient Method for the Determination of Porcine Islet Equivalents Without Counting and Sizing Individual Islets", Cell Transplant. 2003; 12(1: 33-41)

### Biochemische Analyse des Pankreasgewebe und der gereinigten Inseln

Ungefähr 50 mg des nativen Pankreasgewebe aus dem Zwölffingerdarmlappen wurde in HBSS homogenisiert, filtriert und anschließend hinsichtlich des Protein- und Insulingehalts sowie hinsichtlich der Amylaseaktivität untersucht. Auf ähnliche Weise wurde eine Fraktion der Insel-Präparation in HBSS aufgelöst, homogenisiert, filtriert und entsprechend analysiert. Ferner wurde eine Fraktion der gereinigten Inseln hinsichtlich einer Glukose-Insulin-Sekretion getestet.

### Insel-Zellendissoziierung

Die isolierten Inseln wurden in Einzelzell-Suspensionen dissoziiert, und zwar in Aliquots von Insel-Äquivalenten (ungefähr 1.000 bis 1.500) (IEQ) in einer 1 ml Acutase-Lösung (PAA Laboratories, Pasching, Österreich) bei 37°C 15 Minuten lang inkubiert und anschließend durch vorsichtiges Auf- und Abpipetieren dispergiert.

### Bestimmung_ der in den Fraktionen enthaltenen Beta-Zellen durch Fluoreszenzmarkierung mit Newport Green

Die Einzell-Suspensionen wurden mit 1 µM Newport Green PDX Acetoxymethylether (NG; Molecular Probes) 30 Minuten lang bei 37°C in PBS ohne Ca²⁺ und Mg²⁺ inkubiert. Durch Newport Green können Zelluntereinheiten gemäß dem Zinkgehalt bestimmt werden. Nach dem Waschen wurden die Zellen mit 7-Aminoactinomycin D(7-AAD; Molecular Probes) gefärbt, welches an DNA bindet, wenn die Zellmembranpermeabilität nach dem Zelltod verändert wird. Die Zellsuspensionen wurden unter Verwendung eines FACSort Cytometers (Becton Dickinson) mit der ZellQuest Software analysiert.

### Ergebnisse

Der Zwölffingerdarmlappen aller Bauchspeicheldrüsen besaß einen ähnlichen Amylasegehalt in allen Präparationen, jedoch variierte der extrahierbare Insulingehalt in einem breiten Bereich. Alle Bauchspeicheldrüsen (Milzlappen) waren anatomisch intakt und trugen nach einer manuellen Spritzenladung eine ähnliche Menge an Liberase. Die Dauer des Verdaus war ähnlich, ebenso wie die Menge an unverdautem Gewebe nach gründlicher Kammer-Elution.

Dies zeigt also, dass der Verdau der Pankreas-Endabschnitte auf eine standardisierte und reproduzierbare Art und Weise durchgeführt werden kann (siehe nachstehende Tabelle 1). Bei Verwendung des geschlossenen Systems ist es allerdings nicht möglich, die Menge der isolierten Inseln zu analysieren, da das Gewebe direkt in den Beutel des COBE2991 übertragen wird.

Nachstehend bedeuten in der Tabelle 1: Gewicht: abgeschnittener Pankreasendabschnitt; Insulingehalt und Amylaseaktivität sind pro mg Protein angegeben; Geladen: Gewichtzunahme nach Injizierung von 150 ml Liberase PI (1,5 mg/gP); Zeit: Dauer des kontinuierlichen Verdauens/Filtrierens; Rest: in der Kammer verbleibendes Gewebe nach Elution mit insgesamt 3 Litern HBSS, ausgedrückt in Prozent Pankreas nach Liberase-Infusion.

**Tabelle 1: Pankreas-Verdau**

| Präp.-Nr. | Gewicht (g) | Insulin (µg/mg) | Amylase (U/mg) | Geladen (g) | Zeit (min) | Rest (%) |
|---|---|---|---|---|---|---|
| 1 | 100 | 0,36 | 672 | 108 | 29 | 10 |
| 2 | 110 | 1,02 | 2420 | 106 | 25 | 21 |
| 3 | 86 | 0,02 | 5416 | 94 | 25 | 17 |
| 4 | 78 | 0,06 | 6394 | 104 | 37 | 6 |
| 5 | 78 | 0,07 | 1044 | 108 | 34 | 14 |
| 6 | 80 | 1,70 | 1195 | 88 | 32 | 15 |
| M | 89 | 0,54 | 2857 | 101 | 30 | 14 |
| SD | 13 | 0,68 | 2452 | 8 | 5 | 5 |

| | | | | | | |
|---|---|---|---|---|---|---|
| M: Mittelwert; SD = Standardabweichung | | | | | | |

In einem Kontrollexperiment, das mit einem klassischen offenen Setup durchgeführt wurde, wurden insgesamt 821.207 ± 424.314 IEQ oder 9.426 ± 4.585 IEQgP nach der Isolierung detektiert (Mittelwert ± SD in vier Präparationen).

Zur Detektion der gereinigten Inseln nach der diskontinuierlichen UW/OptiPrep-Dichtegradienten-Zentrifugation wurden alle Aliquots von 20 ml gesammelt und hinsichtlich Insulin gefärbt. Die gereinigten Inseln wurden jeweils in der Fraktion mit Nummer 6 beobachtet, wobei sehr kleine Inseln in den benachbarten Fraktionen beobachtet wurden. Im Vergleich zu dem nativen Organ wurde eine beinahe 200-fache Akkumulation an Insulin pro mg Protein in der Insel-Fraktion beobachtet, kombiniert mit einem 45-fachen Abfall der Amylaseaktivität (siehe die nachstehende Tabelle 2).

**Tabelle 2: Insel-Reinigung**

| Präp.-Nr. | IEQ (Anzahl) | IEQ/gP (Anzahl) | Insulin (U) | Insulin (µg/mg) | Amylase (U/mg) |
|---|---|---|---|---|---|
| 1 | 134884 | 1348 | **94** | 72 | 5 |
| 2 | 195655 | 1778 | **63** | 78 | 167 |
| 3 | 87732 | 1020 | **13** | 41 | 4 |
| 4 | 76040 | 975 | **14** | 10 | 93 |
| 5 | 98226 | 1259 | **200** | 349 | 106 |
| 6 | 108712 | 1359 | **166** | 21 | 5 |
| M | 116875 | 1290 | **92** | 95 | 63 |
| SD | 43501 | 290 | **78** | 127 | 69 |

| | | | | | |
|---|---|---|---|---|---|
| M: Mittelwert; SD = Standardabweichung | | | | | |

In der Tabelle 2 stellen die Insel-Äquivalente (IEQ) das Volumen einer idealen Insel mit einem Durchmesser von 150 µm dar. IEQgP stellt IEQ pro Gramm Pankreas-Gewebe dar. Der Insulingehalt in der Insel-Fraktion ist in Units angegeben (1 U: 40 µg Insulin). Der Insulingehalt (µg) und die Amylaseaktivität (U) sind ebenfalls pro mg Protein angegeben.

4/6 der Präparationen zeigten einen positiven Stimulationsindex in Stimulationsexperimenten mit statischer Glukose (Daten nicht gezeigt).

Eine beispielhafte Präparation enthielt gereinigte Inseln, sowie keine sichtbaren Verunreinigungen durch exokrines Gewebe. Die gemessene Amylaseaktivität bedeutete eine Reinheit von größer als 99 %. Zusätzlich zu den isolierten Inseln enthielt diese Präparation Gefäßgewebefragmente.

Diese Insel-Zellpräparation wurde durch FACS-Analysen untersucht. Die Beta-Zellen wurden durch Färben mit NG identifiziert, und die lebenden Zellen wurden nach einer 7-AAD-Inkubation identifiziert. Durch das 7-AAD-Färben wurde eine bestimmte Fraktion toter Zellen identifiziert, und zwar durch Vergleichen mit dem schwachen Fluoreszenzsignal der lebenden Zellen in Gegenwart von 7-AAD. Bei dieser Präparation wurde eine Fraktion von ungefähr 67 % lebende Zellen in der Gesamtpräparation identifiziert. Das Hinzufügen von Newport Green (NG) erhöhte das Fluoreszenzsignal 10- bis 300-fach im Vergleich zu dem autofluoreszierenden Signal ungefärbter Zellen. Auch hier wiederum konnte eine Fraktion von ungefähr 67 % aller Zellen als NGpositiv identifiziert werden. Durch eine kombinierte Analyse von NG-positiven und 7-AAD-negariven Zellen konnte eine Population von ungefähr 40 % lebender Beta-Zellen identifiziert werden (Daten nicht gezeigt).

Die Analyse einer anderen Präparation erzielte eine Beta-Zell-Fraktion von 57 % (Daten nicht gezeigt), was darauf hindeutet, dass in unterschiedlichen Präparationen ein ähnlicher Prozentsatz an Beta-Zellen gefunden werden kann.

### Diskussion

Der COBE2991-Zellprozessor ist für die Prozessierung von Blutzellkonzentraten in einer nichtsterilen Umgebung entwickelt worden. Die Zentrifugierung großer Volumen, die Einfachheit der Generierung von Dichtegradienten und die Möglichkeit, fraktionsweise zu arbeiten, machte den COBE2991 zu einem außerordentlich nützlichen Werkzeug für die Insel-Reinigung. Dennoch wurde bisher der COBE2991 im Stand der Technik nur für den letzten Insel-Reinigungsschritt eingesetzt, wodurch die Inseln wiederholt mit der Umgebungsatmosphäre in Kontakt gekommen wären/sind. Dadurch ist es notwendig, die Präparation der Inseln für klinische Anwendungen in Reinräumen durchzuführen.

Die vorliegende Erfindung zeigt die Nützlichkeit des COBE2991 für die Durchführung einer Insel-Isolierung und -reinigung in einem insgesamt geschlossenen System. Daher ist es möglich, die Insel-Isolierungen auch außerhalb von Reinräumen für klinische Anwendungen durchzuführen, indem das geschlossene Beutelsystem des Zellprozessors auf eine ähnliche Weise genutzt wird, wie für die Herstellung von Blutkomponenten. Obgleich für die anfängliche Organentnahme Reinräume unvermeidbar sind, stellt die erfolgreiche Reinigung großer Mengen an lebenden Inseln einen Beweis für die Durchführbarkeit und Nützlichkeit eines geschlossenen Systems für die Insel-Präparation außerhalb von Reinräumen dar.

Im Vergleich zu dem im Stand der Technik bekannten Apparaturaufbau, wie er beispielsweise von Ricordi et al. beschrieben wird (Ricordi et al., "A Method for the Mass Isolation of Islets From the Adult Pig Pancreas", Diabetes, 1986, 35 (6: 649-653), wird bei dem vorliegenden erfindungsgemäßen Verfahren eine rostfreie Durchflusskühlkammer verwendet, die in direkter Verbindung mit dem COBE-Zentrifugationsbeutel steht. Ferner wird der COBE2991 für ein wiederholtes Verdauen/Zentrifugieren eingesetzt, was zu einer Akkumulierung des verdauten Gewebematerials in dem geschlossenen COBE-Beutel führt. Letztendlich wird die COBE-Mischfunktion für die Resuspendierung des pelletierten Verdaus nach der Isolierung eingesetzt.

Die wiederholte direkte Zentrifugierung des Verdaus beschleunigt das gesamte Herstellungsverfahren, welches nun innerhalb von vier Stunden durch zwei Personen durchgeführt werden kann (bei insgesamt 8 Stunden Präparationszeit). Durch diese Präparationstechnik wird die Sammlung des verdauten Gewebematerials in Wegwerfbehältnissen und die Notwendigkeit einer zweiten Raumzentrifuge vermieden. Aus diesen Gründen kann ein insgesamt geschlossenes System für die Insel-Präparation bereitgestellt werden.

Zusammenfassend lässt sich zeigen, dass mit den vorliegenden Daten dargelegt werden konnte, dass das geschlossene System für die Insel-Isolierung eine äußerst nützliche Präparationstechnik ist, durch welche unter anderem die Notwendigkeit von Reinräumen zur Durchführung der Zellpräparation und Isolierung vermieden werden kann.

## Patentansprüche

1. Verfahren zur *in vitro* Präparation von lebenden Zellen und/oder Zellansammlungen aus einem Gewebe und/oder Organ, mit den folgenden Schritten:
a) Bereitstellen von isoliertem Gewebe- und/oder Organmaterial, das die Zellen von Interesse aufweist,
b) Behandeln des isolierten Gewebe- und/oder Organmaterials in einem Behältnis, um zumindest teilweise zerlegtes und/oder abgebautes Gewebe- und/oder Organmaterial zu gewinnen,
c) Hinzufügen von flüssigem Medium zu dem Gewebe- und/oder Organmaterial aus Schritt b), um zumindest Teile des Gewebe- und/oder Organmaterials aus dem Behältnis in einen Beutel eines Zellprozessors zu eluieren, wobei das Behältnis und der Beutel derart verbunden sind, dass sie ein geschlossenes System bilden,
d) Zentrifugieren des Beutels und Verwerfen des Überstandes zur Gewinnung eines die Zellen von Interesse enthaltenden Konzentrats in dem Zellprozessor,
e) Reinigen des Konzentrats unter Anwendung eines Dichtegradienten in dem Zellprozessor.

2. Verfahren nach Anspruch 1, **dadurch gekennzeichnet, dass** vor Schritt e) die Schritte c) und d) wiederholt werden.

3. Verfahren nach einem der Ansprüche 1 oder 2, **dadurch gekennzeichnet, dass** das Gewebe- und/oder Organmaterial in Schritt b) durch ein Enzym behandelt wird.

4. Verfahren nach einem der Ansprüche 1 bis 3, **dadurch gekennzeichnet, dass** das Behältnis, in welches das Gewebe- und/oder Organmaterial zur Behandlung gemäß Schritt b) gegeben wird, über eine Kühlvorrichtung mit dem Beutel des Zellprozessors verbunden ist.

5. Verfahren nach einem der Ansprüche 1 bis 4, **dadurch gekennzeichnet, dass** als flüssiges Medium ein Medium verwendet wird, das ausgewählt ist aus der Gruppe umfassend isotonische Kochsalzlösung, Ringerlösung, Ringer-Laktat-Lösung, Zellkulturmedien, wie HBSS, HAMS, MEM, Zell- und Organkonservierungslösungen, insbesondere Eurocollins, UW-Lösung, HTK-Lösung, Celsior, Pancoll oder Biocoll, sowie Mischungen davon.

6. Verfahren nach einem der Ansprüche 1 bis 5, **dadurch gekennzeichnet, dass** Gewebe- und/oder Organmaterial bereitgestellt wird, das ausgewählt ist aus der Gruppe umfassend, Pankreas, Leber, Herz, Niere, Lunge, Blase, Ösophagus, Darm, Epidermis, Knorpel.

7. Verfahren nach einem der Ansprüche 1 bis 6, **dadurch gekennzeichnet, dass** Zellen und/oder Zellansammlungen präpariert werden, die ausgewählt sind aus der Gruppe umfassend Langerhans-Inseln/Inselzellen, duktale Pankreaszellen/ Ausführgänge, exokrines Pankreasgewebe; Hepatozyten, Cardiomyozyten, Nierenparenchym, Epitelzellen/Mukosazellen, Chondrozyten, Muskelzellen aus der Darmwand und aus der Blasenwand, Stammzellen oder Vorläuferzellen aus Geweben/Organen.

8. Verfahren nach einem der Ansprüche 1 bis 7, **dadurch gekennzeichnet, dass** der enzymatische Verdau des Gewebe- und/oder Organmaterials in Schritt b) und das Hinzufügen von Medium in Schritt c) in einer Ricordi-Kammer durchgeführt wird.

9. Verfahren nach einem der Ansprüche 1 bis 8, **dadurch gekennzeichnet, dass** zur Dichtegradienten-Reinigung in Schritt e) eine Lösung eingesetzt wird, die ausgewählt ist aus der Gruppe umfassend UW (University of Wisconsin), OptiPrep, Ficoll, Sucrose, Percoll, Albumin, Dextran, Metrizamide, Nycodenz, oder Mischungen davon.

10. Set zur *in vitro* Präparation von lebenden Zellen und/oder Zellansammlungen aus einem Gewebe und/oder Organ, umfassend eine Ricordi-Kammer, eine Kühlvorrichtung und einen Zellprozessor, wobei die Ricordi-Kammer über die Kühlvorrichtung mit dem Zellprozessor derart verbunden ist, dass die Ricordi-Kammer und der Zellprozessor ein geschlossenes System bilden.

11. Verwendung des Sets nach Anspruch 10 zur *in vitro* Präparation von lebenden Zellen aus einem isolierten Gewebe und/oder Organ.

## Claims

1. Method for the *in vitro* preparation of living cells and/or cell clusters from a tissue and/or organ, comprising the following steps:
a) providing of isolated tissue material and/or organ material which comprises the cells of interest,
b) treating, in a container, the isolated tissue and/or organ material to obtain tissue and/or organ material which is at least partially fragmented and/or degraded,
c) adding of a liquid medium to the tissue material and/or organ material from step b), in order to elute parts of the tissue material and/or organ material from the container into a bag of a cell processor, whereby the container and the bag are connected such, that a closed system is formed,
d) centrifuging the bag and discarding the supernatant for recovering, in the cell processor, a concentrate containing the cells of interest,
e) purifying, in the cell processor, the concentrate by using a density gradient.

2. The method of claim 1, **characterized in that** prior to step e) the steps c) and d) are repeated.

3. The method of any of claims 1 or 2, **characterized in that** the tissue material and/or organ material is treated by an enzyme in step b).

4. The method of any of claims 1 to 3, **characterized in that** the container, into which the tissue material and/or organ material is placed for the treatment according to step b), is connected with the bag of the cell processor via a cooling device.

5. The method of any of claims 1 to 4, **characterized in that** a liquid medium is used, which is selected from the group comprising isotonic saline, Ringer's solution, Ringer's-lactate solution, cell culture media, such as HBSS, HAMS, MEM, cell and organ conserving solutions, in particular Euro-Collins, UW-solution, HTK-solution, Celsior, Pancoll or Biocoll, as well as mixtures thereof.

6. The method of any of claims 1 to 5, **characterized in** a tissue material and/or organ material is provided which is selected from the group comprising pancreas, liver, heart, kidney, lung, bladder, esophagus, intestine, epidermis, cartilage.

7. The method of any of claims 1 to 6, **characterized in that** cells and/or cell clusters are prepared, which are selected from the group comprising Langerhans-islets/islet cells, pancreatic duct cells/excretory ducts, exocrine pancreatic tissue; hepatocytes, cardiomyocytes, kidney parenchyma, epithelial cells/mucosa cells, chondrocytes, muscle cells from the intestinal wall and from the bladder wall, stem cells and precursor cells from tissues/organs.

8. The method of any of claims 1 to 7, **characterized in that** the enzymatic degredation of the tissue material and/or organ material in step b) and the adding of medium in step c) are performed in a Ricordi chamber.

9. The method of any of claims 1 to 8, **characterized in that** a solution is applied in step e) for the density gradient purification, which is selected from the group comprising UW (University of Wisconsin), OptiPrep, ficoll, sucrose, percoll, albumin, dextran, metrizamide, Nycodenz, or mixtures thereof.

10. Set for *in vitro* preparation of living cells and/or cell clusters from a tissue and/or organ, comprising a Ricordi chamber, a cooling device and a cell processor, wherein the Ricordi chamber is connected with the cell processor via the cooling device, such, that the Ricordi chamber and the cell processor form a closed system.

11. Use of the set of claim 10 for *in vitro* preparation of living cells from an isolated tissue and/or organ.

## Revendications

1. Procédé de préparation in vitro de cellules vivantes et/ou d'ensembles de cellules provenant d'un tissu et/ou d'un organe, comprenant les étapes consistant à :
a) la mise en place d'un matériau de tissu et/ou d'organe isolé comportant les cellules à examiner ;
b) le traitement du matériau de tissu et/ou d'organe isolé dans un contenant, afin d'obtenir un matériau de tissu et/ou d'organe au moins en partie fragmenté et/ou dégradé,
c) l'ajout d'un milieu liquide au matériau de tissu et/ou d'organe provenant de l'étape b), afin d'éluer au moins des parties du matériau d'organe et/ou de tissu provenant du contenant dans un sac d'un processeur cellulaire, le contenant et le sac étant reliés de telle sorte qu'ils forment un système fermé,
d) la centrifugation du sac et l'élimination du liquide surnageant pour obtenir un concentré contenant les cellules à examiner dans le processeur cellulaire,
e) le nettoyage du concentré en utilisant un gradient de densité dans le processeur cellulaire.

2. Procédé selon la revendication 1, **caractérisé en ce que** les étapes c) et d) sont répétées avant de passer à l'étape (e).

3. Procédé selon l'une quelconque des revendications 1 ou 2, **caractérisé en ce que** le matériau de tissu et/ou d'organe est traité à l'étape b) par un enzyme.

4. Procédé selon l'une quelconque des revendications 1 à 3, **caractérisé en ce que** le contenant dans lequel est délivré le matériau d'organe et/ou de tissu à traiter conformément à l'étape b) est relié au sac du processeur cellulaire par un dispositif de refroidissement.

5. Procédé selon l'une quelconque des revendications 1 à 4, **caractérisé en ce qu'**un milieu choisi dans le groupe comprenant une solution saline isotonique, une solution de Ringer, une solution Ringer lactate, des milieux de culture cellulaire, comme les solutions HBSS, HAMS, MEM, des solutions de conservation cellulaire et organique, en particulier la solution Eurocollins, la solution UW, la solution HTK, Celsior, Pancoll ou Biancoll, et leurs mélanges, est utilisé comme milieu liquide.

6. Procédé selon l'une quelconque des revendications 1 à 6, **caractérisé en ce qu'**un matériau de tissu et/ou d'organe choisi dans le groupe comprenant le pancréas, le foie, le coeur, le rein, le poumon, la vessie, l'oesophage, l'intestin, l'épiderme, le cartilage est fourni comme matériau de tissu et/ou d'organe.

7. Procédé selon l'une quelconque des revendications 1 à 6, **caractérisé en ce que** des cellules et/ou des ensembles de cellules choisies dans le groupe comprenant les cellules d'un îlot/d'îlots de Langerhans, les cellules pancréatiques ductales/canaux pancréatiques ductaux, les tissus pancréatiques exocrines, les hépatocytes, les cardiomyocytes, le parenchyme rénal, les cellules épithéliales/les cellules mucoïdes, les cellules cartilagineuses, les cellules musculaires de la paroi intestinale et de la paroi de la vessie, les cellules souches ou les précurseurs des tissus/organes, sont préparées.

8. Procédé selon l'une quelconque des revendications 1 à 7, **caractérisé en ce que** la digestion enzymatique du matériau de tissu et/ou d'organe est effectuée à l'étape b) et l'ajout d'un milieu à l'étape c) est effectué dans une chambre de Ricordi.

9. Procédé selon l'une quelconque des revendications 1 à 8, **caractérisé en ce qu'**une solution choisie dans le groupe comprenant les solutions UW (de l'université du Wisconsin), OptiPrep, Ficoll, sucrose, Percoll, albumine, dextran, métrizamide, nycodenz, ou leurs mélanges est utilisée pour le nettoyage au moyen du gradient de densité à l'étape e).

10. Kit de préparation in vitro de cellules vivantes et/ou d'ensembles de cellules provenant d'un tissu et/ou d'un organe, comprenant une chambre de Ricordi, un dispositif de refroidissement et un processeur cellulaire, la chambre de Ricordi étant reliée au processeur cellulaire par le dispositif de refroidissement, de telle sorte que la chambre de Ricordi et le processeur cellulaire forment un système fermé.

11. Utilisation du kit selon la revendication 10 pour la préparation in vitro de cellules vivantes provenant d'un tissu et/ou organe isolé.
